(19) Europäisches Patentamt
European Patent Office
Office européen des brevets

(11) Publication number: 0 347 064 A1

(12) **EUROPEAN PATENT APPLICATION**

(21) Application number: 89305498.1

(51) Int. Cl.4: C07C 43/11 , C07C 41/03

(22) Date of filing: 05.06.89

(30) Priority: 13.06.88 US 205754
13.06.88 US 205753

(43) Date of publication of application: 20.12.89 Bulletin 89/51

(84) Designated Contracting States: DE FR GB

(71) Applicant: TEXACO DEVELOPMENT CORPORATION
2000 Westchester Avenue
White Plains New York 10650(US)

(72) Inventor: Nieh, Edward Chung Yit
8604 Alverstone Way
Austin Texas 78759(US)

(74) Representative: Brock, Peter William et al
URQUHART-DYKES & LORD 91 Wimpole Street
London W1M 8AH(GB)

(54) **Preparation of nonionic surfactants by oxyalkylation with a magnesium catalyst.**

(57) Oxyalkylated products having a narrow molecular weight range, which are particularly suitable for use as surfactants, can be obtained by the reaction of $C_{2-4}$ alkylene oxides with linear or branched monohydric alcohols or (alkyl)phenols having 6 to 30 carbon atoms in the presence of specific catalysts. These catalysts are magnesium aryloxides or the reaction products of magnesium aryloxides or high molecular weight magnesium alkoxides with phosphorus-containing acids or esters.

EP 0 347 064 A1

# PREPARATION OF NONIONIC SURFACTANTS BY OXYALKYLATION WITH A MAGNESIUM CATALYST

This invention relates to a process for preparation of nonionic surfactants with a narrow range of molecular weight distribution.

This invention relates more specifically to the preparation of improved nonionic surfactant active agents and more particularly, to a process for the oxyalkylation of certain reactive hydrogen compounds to prepare nonionic surfactant active agents wherein the molecular weight distribution is narrow and wherein a novel magnesium-containing catalyst is employed to produce the nonionic surfactants.

Low molecular weight condensation products of a reactive hydrogen compound, e.g. an alcohol, with an alkylene oxide, particularly ethylene oxide, or with mixtures of alkylene oxides, such as ethylene and propylene oxides, are well known and for a long time have been prepared commercially for use in detergents, cleansing agents, dry cleaning materials, wetting and emulsifying agents and the like. These products are conventionally produced by reacting the reactive hydrogen compound with the alkylene oxide in the presence of a strongly alkaline or acidic catalyst. Such preparative procedures result in the production of a mixture of relatively low molecular weight (up to 2000) species containing a number of alcohol derivatives having different molecular proportions of alkoxylate. Thus, the reaction products generally obtained are, in reality, a mixture of derivatives of the alcohol moiety containing different molecular proportions of alkylene oxide units, i.e., having varying molar ratios of alcohol to alkylene oxide, and a wide range of molecular weights, as well as having a certain proportion of unreacted alcohol. Moreover, it is well known that the conventional designation of the number of alkylene oxide units present per molecule of an alcohol alkoxylate is a designation of the average number of aklylene oxide units per molecule, and that a substantial proportion of the alcohol alkoxylates present are present as alcohol alkoxylates having greater and lesser numbers of alkylene oxide units than the actual average value would indicate. Such designation of such products is well understood in the art, and will be employed herein consistent with its well understood meaning.

It is generally desirable to restrict, i.e. control, the breadth of the molecular weight distribution of the mixture as far as possible to adjacent analogues of the desired product, since, as is well known, the number of moles of alkylene oxide in the reaction product is a major factor in determining the properties of such products, but as a matter of course it is quite difficult to control the molecular weight distribution. Acidic catalysts tend to give a narrower molecular distribution than alkaline catalysts, but, unfortunately, also contribute to the formation of undesired by-products. Thus, alkaline catalysts, which are typically strong bases, such as alkali metal hydroxides and alcoholates, are generally used as the more efficient type of oxyalkylation catalyst, but the molecular distribution of the products will be wider with a greater proportion of lower and higher molecular weight species and smaller amounts of the species with the desired number of moles of alkylene oxide per mole of alcohol. For example, an 8-mole ethylene oxide (EO) 1-dodecanol adduct will contain not only the 8-mole EO adduct itself but also lower mole adducts and higher mole adducts. Lower mole adducts in the product mixture will range down to the 1-mole adduct, and higher adducts will extend up to 14 or 15 and even higher. The molecular weight distribution is a measure of the relative amounts of the various adducts in the product mixture and can be represented by a generally bell-shaped curve when the amount of each adduct species is plotted against the number of moles of epoxide, or by a description of the relative amount of each individual adduct. When the molecular weight distribution is characterized by a bell-shaped curve, a narrower distribution gives a sharper curve which is relatively higher at the middle and lower at the ends, while a broader distribution would give a curve whose middle portion was relatively lower, and whose ends were relatively higher, which is not desirable.

Heretofore, several methods have been suggested for providing reaction products of an active hydrogen compound, e.g. alcohol, and epoxides which have a narrower range of molecular weights and molecular distribution of the epoxide units, and/or which reduce or eliminate the production of undesirable poly-(alkylene glycol) and cyclic and straight chain ether by-products. For example, US-A-4112231 discloses that certain neutral inorganic fluoborate and perchlorate salts will catalyze the reaction of epoxides with active hydrogen compounds to give products having a more limited range of molecular species and a larger proportion of desired molecular species. In US-A-3682849 improved ethoxylated derivatives of $C_{11}$ -$C_{18}$ alcohols are prepared by using vapor phase separation techniques to remove unreacted alcohol and lower ethoxylates from the ethoxylate mixture prepared by conventional methods. US-A-2870220 discloses a two-stage process for preparing monoalkyl ethers of ethylene glycol and polyethylene glycols of more restricted molecular-weight range wherein an alkanol and ethylene oxide are reacted in the presence of an acidic catalyst during the first stage, and then in the second stage after removal of acid catalyst and unreacted alkanol, the mixture is reacted with ethylene oxide in the presence of an alkali metal alcoholate of the initial

alkanol US-A-3972948 discloses a method of preparing mono- and poly-glycol ethers substantially free of undesired alkylene glycol by-products, which involves heating an alkylene oxide and an alcohol in the presence of a catalyst containing alkali or alkaline earth cations, some or all of the catalyst being an anhydrous high boiling liquid residue prepared by concentrating the liquid residue from the same or different etherification processes after removal of the glycol ether product from the reaction mixture. None of the above-described processes and special catalysts disclosed in the art, however, are completely satisfactory for preparing a product with a desired molecular distribution, in that they generally require multi-stage procedures or special acid-resistant equipment, may form undesirable by-products or simply do not provide sufficient control over the molecular weight distribution to be of a satisfactory nature. Thus, it would be highly desirable to develop a process where in the reaction of an alkylene oxide (epoxide) with an alcohol could be more readily carried out to give surfactant products that have a relatively narrow molecular weight distribution of analogue species and contain only small amounts, at most, of undesirable poly-(alkylene glycol) and ether by-products.

Several patents are concerned with the preparation and advantages of nonionic surfactant products having a narrower molecular weight distribution. For example, US-A-4239917 discloses the use of a class of basic barium materials as catalysts in the preparation of reaction products of alcohols and ethylene oxide so as to provide a product with a narrow, high mole adduct distribution while providing relatively low levels of undesirable by-products and unreacted free alcohol. The molecular weight distribution factor of the products produced during the oxyalkylation reaction is discussed at length and the differences in the molecular weight distribution of reaction products prepared with conventional alkali metal catalysts, such as sodium hydroxide, and those prepared using a barium catalyst of the invention is shown by graphical representations. The patent also shows that other alkaline earth metal materials, such as calcium hydroxide, magnesium oxide, and strontium hydroxide, were ineffective as catalysts for the oxyalkylation reaction. This demonstrates that significant differences exist in catalytic effectiveness, even between the various alkaline earth metals and not only between the barium catalysts of the invention and alkali metal hydroxides.

Further, US-A-4210764 and -4223164 are concerned with the problem of the molecular weight distribution of products prepared by oxyalkylation of alcohols using conventional alkaline catalysts, and are directed to overcoming an induction period problem frequently observed when employing barium-containing catalysts, such as those disclosed in US-A-4239917. These patents suggest the use of various phenols as a promoter for the barium-containing catalyst to overcome the induction period difficulty, and US-A-4223164 discloses that with such promoters certain basic strontium materials may also be employed as a catalyst for the oxyalkylation reaction.

US-A-4453022 describes a process for preparing nonionic surfactants having a narrow molecular weight distribution by the use of a basic of calcium and/or strontium hydroxide, alkoxide or phenoxide, and an oxyalkylation catalyst promoter.

US-A-4721817 describes alkylene oxide adducts of higher alkanols characterized by relatively narrow range distribution by the use of a catalyst which combines one or more phosphorus containing acids with one or more aluminium alcoholates or phenolates.

EP-A-0082569 shows that alkanol-soluble basic compounds of magnesium (which could include magnesium alkoxide) are not effective catalysts, but can be activated by alcohol ethoxylates.

There remains, however, a problem of providing effective catalysts that will be effective at producing a narrow molecular weight range oxyalkylation product.

This invention provides an improved method for making narrow range oxyalkylation products using novel catalysts containing magnesium.

In its broadest aspect, this invention provides a method for the oxyalkylation of a linear or branched alcohol or alkyl phenol having 6 to 30 carbon atoms with an alkylene oxide having 2 to 4 carbon atoms by reacting the alcohol and alkylene oxide in the presence of a catalyst. According to the invention, the catalyst is a magnesium aryloxide or the reaction product of a magnesium aryloxide or high molecular weight alkoxide with a phosphorus-containing acid or ester.

Methods for the production of the catalysts used in accordance with the invention will be set out below in greater detail.

In accordance with one embodiment of the present invention, the catalyst is a magnesium aryloxide that may for example be produced from the reaction of magnesium and a phenolic compound. Alternatively, a magnesium alkoxide of the alkanol may be generated in situ, for example, from the reaction of magnesium and a mixture of high molecular weight alcohol(s) and low molecular weight alcohol(s), with the subsequent removal of the low molecular weight alcohols, or simply by mixing a high molecular weight alkanol with magnesium and low molecular weight alcohol(s), with subsequent removal of the low molecular weight alcohol(s). The resulting magnesium-containing compound may then be reacted with a phenolic compound

to produce the catalyst used in this embodiment of the invention.

In accordance with another embodiment of the present invention, the catalyst is produced from the reaction of an alkaline magnesium compound and a phosphorus-containing acid or ester. The alkaline magnesium component is a magnesium alkoxide or aryloxide. The alkaline magnesium component may be generated in situ, for example, by mixing a high molecular weight alkanol with magnesium and low molecular weight alcohol(s) and the subsequent removal of the low molecular weight alcohol(s). Alternatively, the alkaline magnesium component can be formed by mixing a high molecular weight alkanol or a phenol, with a magnesium alkoxide of low molecular weight alcohol(s) and the subsequent removal of the low molecular weight alcohol(s).

The resulting magnesium-containing compound is then reacted with the phosphorus compound, for example with phosphoric acid, to produce the catalyst.

The process of the invention comprises reacting at least one monohydric primary alcohol having 6 to 30 carbon atoms, more preferably 10 to 24 carbon atoms, and especially preferably a linear alkyl primary alcohol with an alkylene oxide having 2 to 4 carbon atoms in the presence of the catalyst.

The reaction may be conducted in a conventional manner, that is, the reactive hydrogen compound and the oxyalkylation catalyst are placed in a reactor, the selected alkylene oxide is added to the reaction mixture until the desired number of moles have been reacted with the reactive hydrogen compound and the product is removed from the reactor and neutralized. The reaction may be conducted in the presence of a solvent, but usually a solvent is not necessary. The process may be batchwise or continuous.

The temperature at which the reaction proceeds is not especially critical, and generally depends upon the desired rate of reaction and sound engineering practices. However, a temperature between 80 and 260°C is usually acceptable with a temperature between 120 and 200°C being preferred. The pressure of the reaction is also not especially critical, although the use of ethylene oxide and/or propylene oxide as the alkylene oxide usually requires a pressurized reactor. In general the pressure may be between 20 and 200 psig (235 and 1480 KPa).

The reaction product may be neutralized and the catalyst residue removed by any conventional technique known to those skilled in the art.

Alcohols which are suitable for the practice of the invention are monohydric primary or secondary alcohols and alkyl phenols having 6 to 30 carbon atoms, and especially useful are linear and branched alkyl primary alcohols of 8 to 24 carbon atoms.

Exemplary of such alcohols are those derived by hydrogenation of natural fats and oils, such as CO and TA alcohols, trademark of Proctor and Gamble Co., such as CO-1214N alcohol, CO-1618 alcohol, and TA 1618 alcohol; and ADOL alcohols, trademark of Ashland Oil Co., such as ADOL 54 alcohol, ADOL 61 alcohol, ADOL 64 alcohol, ADOL 60 alcohol and ADOL 66 alcohol. Alcohols produced by Ziegler chemistry can also be alkoxylated. Examples of these alcohols are ALFOL alcohols trademarks of Continental Oil Co., such as ALFOL 1012 alcohol, ALFOL 1214 alcohol, ALFOL 1412 alcohol, ALFOL 1618 alcohol, or ALFOL 1620 alcohol; and EPAL alcohols, trademark of Ethyl Chemical Co., such as EPAL 1012 alcohol, EPAL 1214 alcohol, or EPAL 1418 alcohol. The invention is extremely useful for oxo alcohols (hydroformylation products from olefins). Examples of such alcohols are NEODOL alcohol, trademark of Shell Oil Co., such as NEODOL 23 alcohol, NEODOL 25 alcohol, or NEODOL 1418 alcohol; TERGITOL-L, trademark of Union Carbide Corp., such as TERGITOL-L 125 alcohol; LIAL alcohols, trademark of Liquichimica Co. such as LIAL 125; and isodecyl and tridecyl alcohols. Guebet alcohols can also be ethoxylated. Representative examples of these alcohols are STANDAMUL alcohols, trademark of Henkel Chemical Co., such as STANDAMUL GT-12 alcohol, STANDAMUL GT-16 alcohol, STANDAMUL GT-20 alcohols, and STANDAMUL GT-1620 alcohol. Secondary alcohols can also be used, such as TERGITOL 15 alcohol, trademark of Union Carbide Corp.

Generally, useable alcohols include 1-decanol; 1-undecanol; 1-dodecanol; 1-tricecanol; 1-tetradecanol; 1-pentadecanol; 1-hexadecanol; 1-heptadecanol; 1-octadecanol; 1-nonadecanol; 1-eicosanol; 1-dicosanol; 2-methyl-1-undecanol; 2-propyl-1-nonanol; 2-butyl-1-octanol; 2-methyl-1-tridecanol; 2-ethyl-1-dodecanol; 2-propyl-1-undecanol; 2-butyl-1-decanol; 2-pentyl-1-nonanol; 2-hexyl-1-octanol; 2-methyl-1-pentadecanol; 2-ethyl-1-tetradecanol; 2-propyl-1-tridecanol; 2-butyl-1-dodecanol; 2-pentyl-1-undecanol; 2-hexyl-1-decanol; 2-heptyl-1-decanol; 2-hexyl-1-nonanol; 2-octyl-1-octanol; 2-methyl-1-heptadecanol; 2-ethyl-1-hexadecanol; 2-propyl-1-pentadecanol; 2-butyl-1-tetradecanol; 1-pentyl-1-tridecanol; 2-hexyl-1-dodecanol; 2-octyl-1-decanol; 2-nonyl-1-nonanol; 2-dodecanol; 3-dodecanol; 4-dodecanol; 5-dodecanol; 6-dodecanol; 2-tetradecanol; 3-tetradecanol; 4-tetradecanol; 5-tetradecanol; 6-tetradecanol; tetradecanol; 7-tetradecanol; 2-hexadecanol; 3-hexadecanol; 5-hexadecanol; 6-hexadecanol; 7-hexadecanol; 8-hexadecanol; 2-octadecanol; 3-octadecanol; 4-octadecanol; 5-octadecanol; 6-octadecanol; 7-octadecanol; 8-octadecanol; 9-octadecanol;9-octadecanol-1; 2,4,6-trimethyl-1-heptanol; 2,4,6,8-tetramethyl-1-nonanol; 3,5,5-trimethyl-1-hexanol; 3,5,5,7,7-pentamethyl-1-octanol; 3-butyl-1-nonanol; 3-butyl-1-undecanol; 3-hexyl-1-undecanol; 3-hexyl-1-tridecanol; 3-octyl-1-tridecanol; 2-methyl-2-undecanol; 3-methyl-3-undecanol; 4-methyl-4-undecanol; 2-methyl-2-tridecanol; 3-methyl-2-tri-decanol; 4-methyl-3-tridecanol; 4-methyl-4-tridecanol; 3-ethyl-3-decanol; 3-ethyl-3-dodecanol; 2,4,6,8-tetramethyl-2-nonanol; 2-methyl-3-undecanol; 2-methyl-4-undecanol; 4-methyl-2-undecanol; 5-methyl-2-undecanol; 4-ethyl-2-decanol; and 4-ethyl-3-decanol.

Also employable as the reactive hydrogen compound are difunctional propylene oxide polymers having a molecular weight of 1000 to 5000, and preferably 1700 to 4100 made with the same catalyst as disclosed herein. The propylene oxide polymers having a molecular weight of 1000 to 5000 contain from 17 to 86 oxypropylene units in the molecule. These compounds are well known, being generally obtained by polymerization of propylene oxide or by the addition of propylene oxide to lower molecular compounds with 2 to 6 carbon atoms containing at least 2 reactive hydrogen atoms.

Alkylene oxides which may be employed in accordance with the invention include those alkylene oxides having 2 to 4 carbon atoms and include, for example, ethylene oxide, 1,2-propylene oxide, and butylene oxides such as 1,2-butylene oxide, and mixtures thereof. The number of moles of alkylene oxide employed according to the present invention may vary widely depending on the reactive hydrogen compound from which adducts are to be formed and the particular application for which the surface active agent is to be employed. In general, between 2 and 80 moles, or even more, of alkylene oxide may be employed per mole of reactive hydrogen compound, with greater molar ratios being employed if higher molecular weight products are desired. Insofar as propylene oxide and/or butylene oxide are used in combination with ethylene oxide the molar ratio of ethylene oxide to propylene oxide or butylene oxide may be between 100:1 and 3:1, preferably between 50:1 and 5:1.

In the process of this invention, the reaction of an alkylene oxide with a reactive hydrogen compound is carried out in the presence of an oxyalkylation catalyst comprising a product produced from the reaction of an alkaline magnesium compound and a phosphorus-containing acid or ester. More specifically, the alkaline magnesium component is the magnesium alkoxide of the alkanol or magnesium phenoxide. The alkaline magnesium component can be generated in situ by various means, for example, by mixing the high molecular weight alkanol or phenol with magnesium alkoxide of a low molecular weight alcohol and the subsequent removal of the low molecular weight alcohol, or by reacting magnesium halide with sodium alkoxide of the high molecular weight alcohol, or with sodium aryloxide and the subsequent removal of sodium halide, or by reacting the high molecular weight alcohol or phenol with alkaline magnesium compounds such as diethyl magnesium, dicyclopentadienyl magnesium, magnesium ammoniate, magnesium amide and magnesium thiophenolate.

When a phenolic compound is used it can be selected from phenol and alkyl substituted phenols, for example, preferred are nonylphenol, dodecylphenol and dinonylphenol.

The phosphorus compounds may be selected from orthophosphoric acid, pyrophosphoric acid, or an alkyl- or aryl-substituted phosphonic acid or phosphinic acid. The $C_1$ to $C_4$ alkyl esters of the above disclosed acids are also useful.

The phosphorus-containing catalyst is generated in situ by adding the phosphorus compound to alkanol containing the magnesium alkoxide or aryloxide. The preferred magnesium to phosphorus ratio is from 2:1 to 1:1.

The reaction scheme below shows a general method of making a catalyst suitable for use according to this invention. The structures shown are believed to be accurate providing a stoichiometric quantity of ROH is available. If less than a stoichiometric quantity of ROH is present, then the catalyst exists in the form of a mixture of $Mg(OR)_2$, $Mg(OCH_3)_2$ and $CH_3$ O-Mg-OR. The scheme shown is for illustration of the method for preparing a catalyst of the invention.

<u>Route 1</u>

$Mg + ROH + CH_3OH \rightarrow Mg(OR)_2 + CH_3OH\uparrow$

where ROH is a phenolic compound.

<u>Route 2</u>

$Mg + R'OH + CH_3OH \rightarrow Mg(OR')_2 + CH_3OH$

(a) $Mg(OR')_2 + ROH \rightarrow Mg(OR)_2$

where R′ is $C_6$ to $C_{30}$ alkyl and

R is phenolic.

Formation of phosphorus-containing catalyst:

$Mg(OR)_2$ + $H_3PO_4.H_2O$

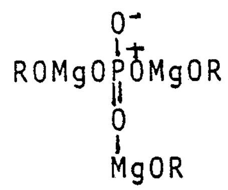

The above reaction schemes show the use of methanol and phosphoric acid. It is of course acceptable to use other low molecular weight alcohols such as ethanol and isopropanol, for example and also above disclosed phosphorus acids and esters. The useful high molecular weight alcohols shown as ROH in reaction (1) are the same as those described above as the reactive hydrogen compounds.

The catalysts used according to the invention produce narrow range alkoxylates that contain smaller amounts of oil-soluble oligomers and smaller amounts of water-soluble oligomers, thus enhancing their usefulness. The peak of molecular weight distribution is especially sharp. As the data will show, this invention provides products containing less of the unreacted alcohol and 1 to 2 mole adducts of alkylene oxide that affect the performance of the product.

Example 1

Magnesium nonylphenoxide was prepared by reacting magnesium turnings (52 grams) with methanol (100 grams) in a three-liter three-neck flask fitted with stirrer, condenser, thermometer and nitrogen inlet. Magnesium turnings were added in 5-gram portions every 10 minutes and the exothermic reaction was controlled at 35 to 45° C with the aid of an ice water bath. When all the magnesium was reacted, nonylphenol (880 grams) was added. The resulting solution contained 2.7% soluble magnesium according to atomic absorption analysis.

Example 2

Magnesium methoxide was prepared by reacting magnesium turnings (60 grams) with anhydrous methanol (1000 grams) in a 2-liter three-neck flask fitted with stirrer, condenser, thermometer and nitrogen inlet. Magnesium turnings were added in 6-gram portions every ten to twelve minutes. Occasionally an ice water bath was used to control reaction temperature in the 35 to 45° C range. Crystalline magnesium methoxide was obtained after the nearly clear supersaturated magnesium solution in methanol was allowed to stand overnight. The solubility of magnesium methoxide in methanol was estimated to be 1000 ppm by atomic absorption analysis of the mother liquor. Crystalline magnesium methoxide was collected and dried in a vacuum oven at 50° C and 20 mm Hg pressure for several hours.

Example 3

The preferred magnesium alkoxide catalyst concentrate was prepared by reacting magnesium turnings (82 gram) with a mixture of anhydrous methanol (920 grams) and EPAL 1214 alcohol (1000 grams, a mixture of dodecanol and tetradecanol, average molecular weight 197, available from Ethyl Corp.) in a three-liter three-neck flask fitted with stirrer, condenser, thermometer and nitrogen inlet. The magnesium turnings were added in small portions every 10 to 12 minute periods. Occasionally, an ice water bath was used to control reaction temperature at 35 to 45° C. When all the magnesium turnings were reacted, the magnesium alkoxide catalyst concentrate remained as a stable clear solution.

Example 4

By a procedure similar to Example 3, a 4.0 weight percent magnesium catalyst concentrate was prepared from magnesium turnings (82 grams), EPAL 1214 alcohol (1000 grams) and methanol (920 grams).

Example 5

To a clean, dried 305 liter kettle was charged EPAL 1214 alcohol (1870 grams) and magnesium catalyst concentrate containing 11.4 gram-atom of Mg in methanol solution. Methanol was stripped from the mixture by heating to 80° C and 20 mm Hg (2.7 KPa) pressure. Then ethylene oxide was added at 165° C and 80 psig (651.6 KPa) until the desired degree of ethoxylation was reached. The product was neutralized with acetic acid (34 grams). Results are given in Table 1.

Example 6

An ethoxylation reaction was conducted in the same manner as described in Example 5 except that ethylene oxide was added at 150° C and 50 psig (444.7 KPa) pressure. Results are given in Table 1.

Example 7

An ethoxylation reaction was conducted in the same manner as described in Example 6, except that the catalyst concentrate contained 22.8 gram-atom of Mg, doubling the quantity used in Example 6. Results are given in Table 1.

Example 8

To a clean and dried 3.5 liter kettle was charged EPAL 1214 alcohol (1834 grams) and a magnesium catalyst concentrate made in the same manner as Example 1 containing 2.3 gram-atom of Mg and 38.7 grams of nonylphenol in methanol. After stripping off methanol, at 80° C and 20 mm Hg (237.9 KPa) pressure, ethoxylation was conducted at 165° C and 80 psi (651.6 KPa) pressure. The product was neutralized with acetic acid. Results are given in Table 1.

Example 9

To a clean and dried 3.5 liter kettle was charged EPAL 1214 alcohol (1870 grams) and a magnesium catalyst concentrate containing 3.06 gram-atom Mg made as in Example 3 and 19 grams of nonylphenol in methanol solution. After stripping off methanol at 80° C and 20 mm Hg (217 KPa) pressure, ethoxylation was conducted in a similar manner to Example 8. The slow reaction was terminated after 5.3 hours. Results are given in Table 1.

Example 10

To a clean and dried 3.5 liter kettle was charged EPAL 1214 alcohol (1870 grams) and a magnesium catalyst concentrate containing 11.4 Mg made as in Example 3 and 49.8 grams of nonylphenol in methanol solution. After stripping off methanol at 80° C and 20 mm Hg (2.7 KPa) pressure, ethoxylation was conducted at 165° C and 80 psi (651.6 KPa) Results are given in Table 1.

Example 11

Example 10 was repeated and results are given in Table 1.

Example 12

Example 6 was repeated using a catalyst concentrate containing 22.8 gram-atom of magnesium catalyst and nonylphenol (100 grams). Results are given in Table 1.

Example 13

Example 6 was repeated using a catalyst concentrate containing 6.8 gram-atom of magnesium and 62.7 grams of nonylphenol Results are given in Table 1.

Example 14

Example 5 was repeated using a catalyst concentrate containing 4.5 gram-atom of magnesium and 83.6 grams of nonylphenol. Results are given in Table 1.

Example 15

Example 5 was repeated using a catalyst concentrate containing 6.8 gram-atom of magnesium and 49.3 grams of dinonylphenol. Results are given in Table 1.

Example 16

Example 10 was repeated using a catalyst concentrate containing 6.9 gram-atom of magnesium and 31.4 grams of 2,6-di-tert-butyl-p-cresol. Results are given in Table 1.

Example 17

An ethoxylation reaction was conducted in a similar manner as in Example 7 except that samples of the product were periodically withdrawn from the reactor and analyzed. Results are given in Table 2.

Example 18 (Comparative)

A calcium 2-methoxyethoxide catalyst in 2-methoxyethanol solution was prepared by reacting calcium carbide (60 parts, 80% technical grade) with 2-methoxyethanol (900 parts) under reflux for 3.5 hours. The catalyst solution containing 3.52% calcium was recovered as a clear liquid after filtration.

To a clean and dried 3.5 liter kettle was charged EPAL 1214 alcohol (1870 grams) and the above-prepared catalyst solution (108 grams) After stripping off 2-methoxyethanol at 120 °C and 20 mm Hg (2.7 KPa) the ethoxylation was conducted at 165 °C and 50 psig (444.7 KPa) Results are given in Table 3.

Example 19 (Comparative)

A calcium nonylphenolate catalyst in methanol solution was prepared by reacting calcium carbide, nonylphenol and methanol under reflux for 6 hours. The catalyst solution contained 2.9% of calcium and 32% of nonylphenol was recovered as clear solution after solids were settled.

To a clean and dried 3.5 liter kettle was charged EPAL 1214 alcohol (1870 grams) and the above prepared catalyst solution (125 grams). After stripping off methanol at 120 °C and 20 mm Hg (2.7 KPa) the ethoxylation was conducted at 165 °C and 50 psig (444.7 KPa). Results are given in Table 3.

Example 20 (Comparative)

To a clean and dried 3.5 liter kettle was charged EPAL 1214 alcohol (1870 grams) and strontium hydroxide hexahydrate (25 grams). After stripping off water at 140 °C and 20 mm Hg (2.7 KPa) the mixture was held at 140 °C for one hour. The ethoxylation was conducted at 165 °C and 80 psig (651.6 KPa). Results are given in Table 3.

Example 21 (Comparative)

To a clean and dried 3.5 liter kettle was charged EPAL 1214 alcohol (1870 grams), strontium hydroxide hexahydrate (25 grams) and nonylphenol (20 grams). After stripping off water, the ethoxylation was conducted at 165 °C and 80 psig (651.6 KPa) Results are given in Table 3.

Example 22 (Comparative)

To a clean and dried 3.5 liter kettle was charged EPAL 1214 alcohol (1870 grams) and barium hydroxide monohydrate (18 grams). After stripping off water, the ethoxylation was conducted at 150 °C and 50 psig (444.7 KPa). Results are given in Table 3.

Example 23 (Comparative)

To a clean and dried 3.5 liter kettle was charged EPAL 1214 alcohol (1870 grams), barium hydroxide monohydrate (18 grams) and nonylphenol (9 grams). After stripping off water, the ethoxylation was conducted at 150 °C and 50 psig (444.7 KPa). Results are given in Table 3.

TABLE 1. ETHOXYLATION OF EPAL 1214 ALCOHOL

| EXAMPLES | 5 | 6 | 7 | 8 | 9 | 10 | 11 | 12 | 13 | 14 | 15 | 16 |
|---|---|---|---|---|---|---|---|---|---|---|---|---|
| CATALYST, Mole % | | | | | | | | | | | | |
| Magnesium Alkoxide | 5.00 | 5.00 | 10.00 | 1.00 | 1.40 | 5.00 | 5.00 | 10.00 | 3.00 | 2.00 | 3.00 | 3.00 |
| Nonylphenol | | | | 1.85 | 0.50 | 2.40 | 2.40 | 4.20 | 3.00 | 4.00 | | |
| Dinonylphenol | | | | | | | | | | | 1.50 | |
| Di-t-butyl-p-cresol | | | | | | | | | | | | 1.50 |
| REACTION CONDITIONS | | | | | | | | | | | | |
| Temperature °C | 165 | 150 | 165 | 165 | 165 | 165 | 165 | 165 | 150 | 150 | 165 | 165 |
| Pressure Psig/KPa | 80/651.6 | 50/444.7 | 50/444.7 | 80/651.6 | 80/651.6 | 80/651.6 | 80/651.6 | 80/651.6 | 50/444.7 | 50/444.7 | 80/651.6 | 80/651.6 |
| Addition Time Hr | 5.00 | 4.50 | 4.20 | 4.80 | 5.30 | 2.80 | 3.50 | 3.25 | 3.33 | 4.58 | 4.51 | 4.51 |
| PRODUCT | | | | | | | | | | | | |
| # Ave. MW | 496 | 512 | 495 | 495 | 351 | 483 | 498 | 518 | 463 | 493 | 498 | 498 |
| # Mole EO | 6.79 | 7.15 | 6.79 | 6.79 | 3.50 | 6.50 | 68.40 | 7.29 | 6.14 | 68.30 | 68.40 | 64.40 |
| PEG % | 0.75 | 0.27 | 0.50 | 0.70 | 0.74 | 0.56 | 0.56 | 0.41 | 1.24 | 1.03 | 1.24 | 1.25 |
| Peak +/-2EO Wt% | 66.70 | 64.50 | 66.50 | 64.50 | 70.40 | 70.80 | 67.70 | 65.90 | 68.80 | 66.80 | 66.90 | 67.50 |

TABLE 1. (CONTINUED)

| EXAMPLES | 5 | 6 | 7 | 8 | 9 | 10 | 11 | 12 | 13 | 14 | 15 | 16 |
|---|---|---|---|---|---|---|---|---|---|---|---|---|
| OLIGOMERS | | | | | | | | | | | | |
| 0EO | 0.57 | 0.06 | 0.77 | 1.90 | 4.70 | 0.15 | 0.74 | 0.60 | 0.52 | 0.36 | 0.83 | 0.60 |
| 1EO | 0.62 | 0.41 | 0.36 | 0.77 | 7.79 | 0.48 | 0.10 | 0.14 | 0.73 | 0.46 | 0.17 | 0.30 |
| 2EO | 1.45 | 1.24 | 1.33 | 2.10 | 13.73 | 1.79 | 0.60 | 2.50 | 2.42 | 1.58 | 0.70 | 1.14 |
| 3EO | 3.24 | 3.12 | 3.69 | 3.25 | 16.49 | 4.16 | 1.90 | 5.05 | 5.72 | 3.73 | 2.25 | 3.08 |
| 4EO | 6.11 | 5.88 | 6.42 | 7.27 | 13.57 | 7.95 | 4.36 | 8.32 | 9.97 | 7.04 | 5.30 | 6.45 |
| 5EO | 8.89 | 9.30 | 10.36 | 10.56 | 10.20 | 11.70 | 8.98 | 12.67 | 13.98 | 11.04 | 10.63 | 11.08 |
| 6EO | 11.54 | 12.46 | 13.89 | 13.30 | 7.10 | 15.40 | 14.20 | 14.56 | 16.64 | 14.50 | 14.75 | 14.19 |
| 7EO | 13.31 | 14.74 | 15.79 | 14.76 | 4.41 | 16.62 | 16.93 | 14.77 | 15.38 | 15.17 | 17.33 | 15.32 |
| 8EO | 12.72 | 14.39 | 14.28 | 13.30 | 2.67 | 14.40 | 16.82 | 13.33 | 12.76 | 14.36 | 16.27 | 14.51 |
| 9EO | 9.22 | 12.65 | 12.14 | 9.25 | 1.43 | 12.70 | 14.05 | 10.47 | 9.40 | 11.67 | 12.92 | 12.47 |
| 10EO | 9.48 | 10.85 | 8.77 | 7.13 | 0.67 | 7.61 | 9.82 | 7.20 | 6.04 | 8.50 | 8.77 | 8.89 |
| 11EO | 4.48 | 6.85 | 5.69 | 3.11 | 0.28 | 5.42 | 5.91 | 4.75 | 3.45 | 5.59 | 5.18 | 5.86 |
| 12EO | 3.21 | 4.43 | 3.20 | 1.10 | | 1.41 | 3.16 | 2.68 | 1.75 | 3.11 | 2.69 | 3.20 |
| 13EO | 1.51 | 2.47 | 1.89 | 0.30 | | 0.11 | 1.58 | 1.37 | 0.79 | 1.61 | 1.29 | 1.79 |
| 14EO | | 1.28 | 0.92 | | | | 0.45 | 0.50 | 0.31 | 0.75 | 0.64 | 0.76 |
| 15EO | | 0.53 | 0.36 | | | | 0.26 | 0.17 | 0.09 | 0.26 | 0.18 | 0.32 |
| 16EO | | 0.17 | 0.10 | | | | | | | 0.21 | | |
| 17EO | | | | | | | | | | | | |
| 18EO | | | | | | | | | | | | |
| 19EO | | | | | | | | | | | | |
| 20EO | | | | | | | | | | | | |

TABLE 2. ETHOXYLATION OF EPAL 1214 ALCOHOL

| | | | | | | POISSON DISTRIBUTIONS | | | | |
|---|---|---|---|---|---|---|---|---|---|---|
| EXAMPLE | | | 17 | | | | | | | |
| SAMPLE NO. | 1 | 2 | 3 | 4 | 5 | | | | | |
| CATALYST, Mole % | | | | | | | | | | |
| Magnesium Alkoxide | 5.00 | 5.00 | 5.00 | 5.00 | 5.00 | | | | | |
| Nonylphenol | 2.30 | 2.30 | 2.30 | 2.30 | 2.30 | | | | | |
| REACTION CONDITIONS | | | | | | | | | | |
| Temperature °C | 165 | 165 | 165 | 165 | 165 | | | | | |
| Pressure Psig/KPa | 80/651.6 | 80/651.6 | 80/651.6 | 80/651.6 | 61/520.6 | | | | | |
| Addition Time Hr | 1.83 | 1.83 | 1.90 | 2.00 | 2.50 | | | | | |
| PRODUCT | | | | | | | | | | |
| # Ave. Mw | 303 | 337 | 377 | 401 | 521 | 303 | 337 | 377 | 401 | 521 |
| # Mole EO | 2.40 | 3.20 | 4.10 | 4.64 | 7.36 | 2.40 | 3.20 | 4.10 | 4.64 | 7.36 |
| PEG % | 0.09 | 0.10 | 0.14 | 0.19 | 0.42 | | | | | |
| Peak +/-2EO Wt% | 91.00 | 84.50 | 79.35 | 78.00 | 65.20 | 88.43 | 82.34 | 7.44 | 7.07 | 63.72 |

TABLE 2. (CONTINUED)

| EXAMPLE SAMPLE NO. | 17 1 | 2 | 3 | 4 | 5 | | | | | |
|---|---|---|---|---|---|---|---|---|---|---|
| OLIGOMERS | | | | | | | | | | |
| 0E0 | 5.30 | 2.53 | 1.13 | 0.88 | 0.07 | 5.92 | 2.38 | 0.87 | 0.48 | 0.02 |
| 1E0 | 14.29 | 7.58 | 3.64 | 2.32 | 0.27 | 17.36 | 9.32 | 4.35 | 2.70 | 0.22 |
| 2E0 | 27.23 | 18.48 | 9.94 | 7.40 | 0.87 | 24.61 | 17.62 | 10.53 | 7.39 | 0.94 |
| 3E0 | 26.02 | 23.31 | 17.16 | 12.85 | 2.45 | 22.72 | 21.68 | 16.60 | 13.19 | 2.67 |
| 4E0 | 16.12 | 20.78 | 19.99 | 16.96 | 4.87 | 15.45 | 19.66 | 19.29 | 17.35 | 5.58 |
| 5E0 | 7.47 | 14.28 | 18.57 | 18.16 | 8.28 | 8.29 | 14.06 | 17.67 | 17.99 | 9.17 |
| 6E0 | 2.59 | 7.66 | 13.68 | 15.89 | 11.61 | 3.66 | 8.29 | 13.35 | 15.38 | 12.43 |
| 7E0 | 0.88 | 3.41 | 8.60 | 12.13 | 14.32 | 1.38 | 4.15 | 8.56 | 11.16 | 14.32 |
| 8E0 | | 1.32 | 4.23 | 6.79 | 14.74 | 0.45 | 1.80 | 4.77 | 7.04 | 14.32 |
| 9E0 | | 0.42 | 1.90 | 3.64 | 13.32 | 0.13 | 0.69 | 2.35 | 3.92 | 12.65 |
| 10E0 | | 0.17 | 0.72 | 1.65 | 10.91 | 0.03 | 0 24 | 1.03 | 3.92 | 10.00 |
| 11E0 | | 0.04 | 0.21 | 0.71 | 7.70 | 0.01 | 0.07 | 0.41 | 1.95 | 7.15 |
| 12E0 | | | 0.06 | 0.34 | 5.16 | | 0.02 | 0.15 | 0.88 | 4.67 |
| 13E0 | | | 0.04 | 0.15 | 2.94 | | 0.01 | 0.05 | 0.36 | 2.80 |
| 14E0 | | | | 0.06 | 1.57 | | | 0.02 | 0.14 | 1.56 |
| 15E0 | | | | | 0.65 | | | | 0.05 | 0.81 |
| 16E0 | | | | | 0.23 | | | | 0.02 | 0.39 |
| 17E0 | | | | | | | | | | 0.18 |
| 18E0 | | | | | | | | | | 0.08 |
| 19E0 | | | | | | | | | | 0.03 |
| 20E0 | | | | | | | | | | 0.01 |

TABLE 3. COMPARATIVE EXAMPLES

| EXAMPLE NO. | 9 | 14 | 18 | 19 | 20 | 21 | 22 | 23 | POISSON DISTRIBUTIONS | |
|---|---|---|---|---|---|---|---|---|---|---|
| CATALYST, Mole % | | | | | | | | | | |
| Magnesium Alkoxide | 1.00 | 2.00 | | | | | | | | |
| Calcium Alkoxide | | | 1.00 | 2.90 | | | | | | |
| Strontium Alkoxide | | | | | 1.00 | 1.00 | | | | |
| Barium Alkoxide | | | | | | | 1.00 | 1.00 | | |
| Nonylphenol | 1.85 | 4.00 | 2.00 | | 2.00 | | 2.00 | | | |
| REACTION CONDITIONS | | | | | | | | | | |
| Temperature °C | 165 | 150 | 165 | 140 | 165 | 165 | 150 | 150 | | |
| Pressure Psig/KPa | 80/651.6 | 50/444.7 | 80/651.6 | 50/444.7 | 80/651.6 | 80/651.6 | 50/444.7 | 50/444.7 | | |
| Addition Time Hr | 4.80 | 4.58 | 2.00 | 3.00 | 2.35 | 1.00 | 1.60 | 1.15 | | |
| PRODUCT | | | | | | | | | | |
| # Ave. MW | 495 | 493 | 493 | 502 | 515.00 | 490 | 523 | 511 | | |
| # Mole EO | 6.79 | 68.30 | 6.79 | 6.93 | 7.23 | 6.66 | 7.40 | 7.13 | 7.00 | 7.40 |
| PEG % | 0.70 | 1.03 | 0.22 | 3.50 | 1.23 | 1.50 | 3.11 | 2.02 | | |
| Peak +/-2EO Wt% | 61.17 | 66.80 | 64.70 | 66.34 | 6.18 | 64.40 | 62.90 | 61.93 | 65.20 | 63.69 |

TABLE 3. (CONTINUED)

| EXAMPLE NO. | 9 | 14 | 18 | 19 | 20 | 21 | 22 | 23 | | |
|---|---|---|---|---|---|---|---|---|---|---|
| OLIGOMERS | | | | | | | | | | |
| 0EO | 1.90 | 0.36 | 1.17 | 0.12 | 1.21 | 0.96 | 0.50 | 0.59 | 0.03 | 0.02 |
| 1EO | 0.77 | 0.46 | 0.63 | 0.50 | 0.36 | 0.76 | 0.57 | 0.70 | 0.30 | 0.21 |
| 2EO | 2.10 | 1.58 | 1.71 | 1.63 | 0.70 | 1.97 | 1.19 | 1.48 | 0.13 | 0.91 |
| 3EO | 3.25 | 3.73 | 3.37 | 3.27 | 2.57 | 3.97 | 2.66 | 3.25 | 3.40 | 2.60 |
| 4EO | 7.27 | 7.04 | 6.23 | 6.06 | 5.69 | 6.39 | 4.78 | 5.69 | 6.70 | 5.46 |
| 5EO | 10.56 | 11.04 | 9.73 | 10.36 | 8.52 | 9.27 | 7.41 | 8.39 | 10.60 | 9.02 |
| 6EO | 13.30 | 14.50 | 12.73 | 13.51 | 11.55 | 15.50 | 11.00 | 11.70 | 13.60 | 12.30 |
| 7EO | 14.76 | 15.17 | 14.93 | 15.10 | 13.29 | 13.67 | 13.45 | 13.55 | 14.90 | 14.24 |
| 8EO | 13.30 | 14.36 | 12.62 | 14.72 | 13.73 | 13.56 | 14.24 | 13.91 | 14.20 | 14.32 |
| 9EO | 9.25 | 11.67 | 9.93 | 12.65 | 12.70 | 11.62 | 13.22 | 12.62 | 11.90 | 12.72 |
| 10EO | 7.13 | 8.50 | 6.25 | 9.45 | 10.38 | 8.79 | 11.00 | 10.15 | 8.90 | 10.11 |
| 11EO | 3.11 | 5.59 | 4.02 | 5.83 | 7.77 | 5.94 | 8.23 | 7.40 | 6.10 | 7.27 |
| 12EO | 1.10 | 3.11 | 1.53 | 3.55 | 5.32 | 3.63 | 5.46 | 4.88 | 3.80 | 4.77 |
| 13EO | 0.30 | 1.61 | 0.35 | 1.82 | 3.18 | 2.13 | 3.37 | 2.84 | 2.20 | 2.88 |
| 14EO | | 0.75 | 0.19 | 0.64 | 1.73 | 1.12 | 1.74 | 1.56 | 1.10 | 1.61 |
| 15EO | | 0.26 | 0.07 | 0.34 | 0.78 | 0.68 | 0.82 | 0.75 | 0.30 | 0.84 |
| 16EO | | 0.21 | | 0.24 | 0.38 | | 0.32 | 0.35 | 0.10 | 0.41 |
| 17EO | | | | | | | | 0.15 | | 0.19 |
| 18EO | | | | | | | | | | 0.08 |
| 19EO | | | | | | | | | | 0.03 |
| 20EO | | | | | | | | | | 0.01 |

Example 24

To a clean, dried 3.5 liter kettle was charged EPAL 1214 alcohol (1870 grams) and magnesium methoxide catalyst prepared in Example 2 (24 grams). The kettle was heated to 80 °C at 20 mm Hg (2.7

KPa) pressure in order to strip off methanol. Then, ethylene oxide was added at 165°C and 80 psi (651.6 KPa) until the desired degree of ethoxylation was reached. The product was neutralized with acetic acid (34 grams). Results are given in Table 4.

Example 25

To a clean, dried 3.5 liter kettle was charged EPAL 1214 alcohol (1750 grams) and magnesium catalyst (11.4 gram-atom Mg, introduced as a catalyst concentrate together with EPAL 1214 alcohol, 121 grams, in a methanol solution). After stripping off methanol, ethylene oxide was added in the same amount as described in Example 24. Results are given in Table 4.

Example 26

To a clean and dried 3.5 liter kettle was charged EPAL 1214 alcohol (1686 grams) and magnesium catalyst (11.4 gram-atom Mg, introduced as a catalyst concentrate together with 184 grams of EPAL 1214 alcohol in a methanol solution). After stripping off methanol, ethylene oxide was added at 150°C and 50 psi (444.7 KPa) pressure. The product was neutralized with acetic acid (108 grams). Results are given in Table 4.

Example 27

To a clean and dried 3.5 liter kettle was charged EPAL 1214 alcohol (1586 grams) and magnesium catalyst (22.8 gram-atom Mg, introduced as a catalyst concentrate together with 284 grams of EPAL 1214 alcohol in a methanol solution). After stripping off methanol, ethylene oxide was added at 165°C and 80 psi (651.6 KPa) pressure. The product was neutralized with acetic acid (108 grams). Results are given in Table 4.

Example 28

To a clean and dried 3.5 liter kettle was charged EPAL 1214 alcohol (1764 grams) and magnesium catalyst (6.8 gram-atom Mg, introduced as a catalyst concentrate together with 116 grams of EPAL 1214 alcohol in a methanol solution). After stripping off methanol, at 80°C and 20 mm Hg (2.7 KPa) pressure, concentrated phosphoric acid 85% in water (10.9 grams) was added. The kettle was again evacuated to 20 mm Hg (2.7 KPa) and heated to 120°C in order to stip off water. Ethylene oxide was added at 150°C and 50 psi (444.7 KPa) pressure over a period of 2.75 hours. The product was neutralized with acetic acid (17 grams). Results are given in Table 4 as Example 28A. This Example was repeated and results are given in Table 4 as Example 28B.

Example 29

Ethoxylation of EPAL 1214 alcohol was conducted in the same manner as in Example 28 except that magnesium catalyst 5.0 mole % (11.4 gram-atom Mg) was used in place of 3.0 mole % (6.8 gram Mg). Results are given in Table 4.

Example 30

Ethoxylation of EPAL 1214 alcohol was conducted in the same manner as in Example 28 except that 98% phosphoric acid (9.5 grams) was used in place of 85% phosphoric acid. Results are given in Table 4.

Example 31

Ethoxylation of EPAL 1214 alcohol was conducted in the same manner as in Example 28 except that 97% phosphorous acid (7.8 grams) was used in place of 85% phosphoric acid. Results are given in Table 4.

Example 32

Ethoxylation of EPAL 1214 alcohol was conducted in the same manner as in Example 28 except that phosphorus pentoxide (6.7 grams) was used in place of 85% phosphoric acid. Results are given in Table 4.

To illustrate that the magnesium alkoxide activated by phosphoric acid or phosphorous acid in an ethoxylation reaction produced products that have better oligomer distribution than those made with prior art catalyst, the following Comparative Examples are provided.

Comparative Examples 33 to 36

Ethoxylation of EPAL 1214 alcohol was conducted using magnesium catalyst concentrate and phosphoric acid to produce 2.0, 2.5, 3.5 and 4.0 mole ethoxylates.

Example 37

The ethoxylation of EPAL 1214 alcohol using magnesium catalyst concentrate and phosphorous acid to a 3.5 mole ethoxylate.

The ethoxylation was conducted in the same manner as disclosed in Example 28 except that 97% phosphorous acid (7.8 grams) was used in place of 85% phosphoric acid. Results are given in Table 5.

Example 38

The ethoxylation of EPAL 1214 alcohol using magnesium catalyst concentrate and phenylphosphonic acid.

Ethoxylation of EPAL 1214 alcohol was conducted in the same manner as in Example 28 except that phenylphosphonic acid (22.5 grams) was used in place of 85% phosphoric acid. Results are given in Table 6.

Example 39

The ethoxylation of EPAL 1214 alcohol using magnesium catalyst concentrate and phenylphosphinic acid.

Ethoxylation of EPAL 1214 alcohol was conducted in the same manner as in Example 28 except that phenylphosphinic acid (40 grams) was used in place of 85% phosphoric acid. Results are given in Table 6.

Example 40

The ethoxylation of EPAL 1214 alcohol using magnesium catalyst concentrate and dimethyl phosphite.

Ethoxylation of EPAL 1214 alcohol was conducted in the same manner as in Example 28 except that dimethyl phosphite (15.6 grams) was used in place of 85% phosphoric acid. Results are given in Table 6.

Example 41

The ethoxylation of EPAL 1214 alcohol using magnesium catalyst concentrate and pyrophosphoric acid.

Ethoxylation of EPAL 1214 alcohol was conducted in the same manner as in Example 28 except that pyrophosphoric acid (12.6 grams) was used in place of 85% phosphoric acid. Results are given in Table 6.

Table 7 showing the Poisson distribution is given to illustrate the advantages of the invention. For

example, in some cases alkoxylation products prepared using the phosphorus-containing catalyst have a range of oligomers comparing favourably with the Poisson distribution. For example, compare Example 28B shown on Tables 4 and 5 with the Poisson distribution as well as those shown in the prior art patents.

TABLE 4. ETHOXYLATION OF EPAL 1214 ALCOHOL

| EXAMPLE NO. | 24 | 25 | 26 | 27 | 28A | 28B | 29 | 30 | 31 | 32 |
|---|---|---|---|---|---|---|---|---|---|---|
| CATALYST, Mole % | | | | | | | | | | |
| Magnesium Alkoxide | 5.00 | 5.00 | 10.00 | 10.00 | 3.00 | 3.00 | 5.00 | 3.00 | 3.00 | 3.00 |
| Phosphoric Acid, 85% | | | | | 1.00 | 1.00 | 1.00 | | | |
| Phosphoric Acid, 98% | | | | | | | | 1.00 | | |
| Phosphorous Acid, 97% | | | | | | | | | 1.00 | |
| Phosphorus Pentoxide | | | | | | | | | | 0.50 |
| REACTION CONDITIONS | | | | | | | | | | |
| Temperature °C | 165 | 165 | 150 | 165 | 150 | 150 | 150 | 150 | 150 | 150 |
| Pressure Psig/KPa | 80/651.6 | 80/651.6 | 50/444.7 | 80/651.6 | 50/444.7 | 50/444.7 | 50/444.7 | 50/444.7 | 50/444.7 | 50/444.7 |
| Addition Time Hr | 3.80 | 5.00 | 5.00 | 4.20 | 1.92 | 2.12 | 1.97 | 1.55 | 3.75 | 9.50 |
| PRODUCT | | | | | | | | | | |
| # Ave. MW | 379 | 496 | 512 | 495 | 497 | 492 | 516 | 500 | 501 | 478 |
| # Mole EO | 4.14 | 6.80 | 7.16 | 6.80 | 6.80 | 6.70 | 7.25 | 6.90 | 6.90 | 6.38 |
| PEG % | 0.45 | 0.75 | 0.27 | 0.50 | 3.60 | 2.17 | 1.97 | 1.55 | 1.78 | 1.07 |
| Peak +/-2EO Wt% | 74.14 | 66.70 | 64.50 | 66.50 | 75.20 | 77.60 | 71.60 | 74.50 | 74.01 | 67.90 |

TABLE 4. (CONTINUED)

| EXAMPLE NO. | 24 | 25 | 26 | 27 | 28A | 28B | 29 | 30 | 31 | 32 |
|---|---|---|---|---|---|---|---|---|---|---|
| OLIGOMERS | | | | | | | | | | |
| 0EO | 3.19 | 0.57 | 0.06 | 0.77 | 0.68 | 0.53 | 0.74 | 0.70 | 0.15 | 0.07 |
| 1EO | 3.45 | 0.62 | 0.41 | 0.36 | 0.20 | 0.12 | 0.10 | 0.17 | 0.28 | 0.44 |
| 2EO | 8.38 | 1.45 | 1.24 | 1.33 | 0.63 | 0.64 | 0.60 | 0.65 | 0.79 | 1.38 |
| 3EO | 14.55 | 3.24 | 3.12 | 3.69 | 2.21 | 2.36 | 1.90 | 2.00 | 2.44 | 3.48 |
| 4EO | 18.12 | 6.11 | 5.88 | 6.42 | 5.42 | 5.73 | 4.36 | 5.26 | 5.65 | 6.31 |
| 5EO | 17.58 | 8.89 | 9.30 | 10.36 | 11.08 | 11.66 | 8.98 | 10.45 | 10.70 | 10.44 |
| 6EO | 14.29 | 11.54 | 12.46 | 13.89 | 16.39 | 16.87 | 14.20 | 15.72 | 15.60 | 15.74 |
| 7EO | 9.60 | 13.31 | 14.74 | 15.79 | 18.77 | 19.54 | 16.93 | 18.67 | 18.19 | 14.80 |
| 8EO | 5.45 | 12.72 | 14.39 | 14.28 | 16.58 | 17.52 | 16.82 | 16.82 | 16.69 | 12.43 |
| 9EO | 3.04 | 9.22 | 12.65 | 12.14 | 12.35 | 12.30 | 14.05 | 12.83 | 12.82 | 8.82 |
| 10EO | 1.44 | 4.48 | 10.85 | 8.77 | 7.64 | 7.10 | 9.82 | 8.12 | 8.18 | 5.60 |
| 11EO | 0.66 | 3.21 | 6.85 | 5.69 | 4.12 | 3.47 | 5.91 | 4.48 | 4.54 | 3.16 |
| 12EO | 0.21 | 1.51 | 4.43 | 3.20 | 2.05 | 1.41 | 3.16 | 2.17 | 2.16 | 1.57 |
| 13EO | | | 2.47 | 1.89 | 0.99 | 0.63 | 1.58 | 1.00 | 0.95 | 0.75 |
| 14EO | | | 1.28 | 0.92 | 0.51 | 0.36 | 0.45 | 0.49 | 0.42 | 0.29 |
| 15EO | | | 0.57 | 0.36 | 0.22 | 1.41 | 0.26 | 0.25 | 0.22 | 0.14 |
| 16EO | | | 0.17 | 0.10 | 0.11 | 0.63 | | 0.16 | 0.16 | |
| 17EO | | | | 0.92 | | 0.36 | | | | |
| 18EO | | | | 0.36 | | | | | | |
| 19EO | | | | | | | | | | |
| 20EO | | | | | | | | | | |

TABLE 5: ETHOXYLATION OF DETERGENT ALCOHOLS - COMPARATIVE EXAMPLES

| | | | | | | US-A-4721817 | | | US-A-4453022 | |
|---|---|---|---|---|---|---|---|---|---|---|
| EXAMPLE NO. | 33 | 34 | 35 | 36 | 2 | 3 | 4 | 4 | 33 | 8B |
| CATALYST, Mole % | | | | | | | | | | |
| Mg Alkoxide | 3.00 | 3.00 | 3.00 | 3.00 | | | | | | 3.00 |
| Al Isopropoxide | | | | | 1.30 | 0.065 | 0.065 | | | |
| Ca Alkoxide | | | | | | | | 4.00 | | |
| Sr Alkoxide | | | | | | | | | 2.09 | |
| Phosphoric Acid, 85% | 1.00 | 1.00 | 1.00 | | 0.65 | 0.033 | 0.330 | 1.11 | 0.78 | 1.00 |
| Phosphorous Acid, 97% | | | | 1.00 | | | | | | |
| REACTION CONDITIONS | | | | | | | | | | |
| Temperature °C | 150 | 165 | 150 | 150 | 140 | 140 | 140 | 140 | 140 | 150 |
| Pressure Psig/KPa | 50/444.7 | 50/444.7 | 50/444.7 | 50/444.7 | 30/306.8 | 30/306.8 | 30/306.8 | 60/513.7 | 60/513.7 | 50/444.7 |
| Addition Time Hr | 1.10 | 1.10 | 1.10 | 1.33 | 2.00 | 1.00 | 1.00 | 1.66 | 1.33 | 2.12 |
| PRODUCT | | | | | | | | | | |
| # Mole EO | 2.00 | 2.50 | 3.50 | 3.50 | 3.40 | 2.10 | 2.10 | 7.36 | 6.42 | 6.70 |
| PEG % | 0.45 | 0.75 | 1.54 | 1.24 | 0.60 | 0.90 | 0.40 | ? | ? | 2.17 |
| Peak +/-2EO Wt% | 90.13 | 89.83 | 83.56 | 82.90 | 81.00 | 84.80 | 86.80 | 73.30 | 70.80 | 77.60 |

TABLE 5. (CONTINUED)

| EXAMPLE NO. | 33 | 34 | 35 | 36 | 2 | 3 | 4 | 4 | 33 | 88 |
|---|---|---|---|---|---|---|---|---|---|---|
| OLIGOMERS | | | | | | | | | | |
| 0E0 | 5.32 | 4.78 | 1.59 | 2.30 | 2.90 | 11.40 | 9.70 | 3.20 | 2.20 | 0.53 |
| 1E0 | 14.94 | 8.40 | 3.76 | 2.52 | 4.70 | 16.50 | 18.50 | 0.50 | 1.60 | 0.12 |
| 2E0 | 24.58 | 19.00 | 10.89 | 7.89 | 11.00 | 23.80 | 25.60 | 1.00 | 2.80 | 0.64 |
| 3E0 | 26.22 | 24.67 | 19.24 | 16.57 | 17.50 | 23.40 | 22.80 | 2.30 | 5.70 | 2.36 |
| 4E0 | 16.44 | 20.92 | 21.38 | 21.92 | 21.60 | 14.70 | 13.70 | 5.40 | 10.00 | 11.66 |
| 5E0 | 7.69 | 13.79 | 19.54 | 20.65 | 18.40 | 6.40 | 6.20 | 10.30 | 14.70 | 16.87 |
| 6E0 | 3.24 | 5.63 | 12.40 | 14.95 | 12.50 | 2.20 | 2.40 | 15.80 | 17.20 | 19.54 |
| 7E0 | 1.14 | 1.91 | 6.53 | 8.00 | 6.50 | 0.90 | 0.70 | 18.10 | 16.20 | 17.52 |
| 8E0 | 0.43 | 0.87 | 2.57 | 3.40 | 2.00 | 0.40 | 0.20 | 16.50 | 12.70 | 12.30 |
| 9E0 | | | 1.26 | 1.35 | 1.40 | | | 12.60 | 8.50 | 7.10 |
| 10E0 | | | 0.52 | 0.45 | 0.70 | | | 8.20 | 4.80 | 3.47 |
| 11E0 | | | | | 0.50 | | | 4.30 | 2.40 | 1.41 |
| 12E0 | | | | | 0.20 | | | 1.60 | 1.00 | 0.63 |
| 13E0 | | | | | | | | 0.30 | 0.30 | 0.36 |
| 14E0 | | | | | | | | | | |
| 15E0 | | | | | | | | | | |
| 16E0 | | | | | | | | | | |
| 17E0 | | | | | | | | | | |
| 18E0 | | | | | | | | | | |
| 19E0 | | | | | | | | | | |
| 20E0 | | | | | | | | | | |

TABLE 6.

| ETHOXYOLATION OF EPAL 1214 ALCOHOL | | | | |
|---|---|---|---|---|
| EXAMPLE NO. | 38 | 39 | 40 | 41 |
| CATALYST, Mole % | | | | |
| Magnesium Alkoxide | 3.00 | 3.00 | 3.00 | 3.00 |
| Phenylphosphonic Acid | 1.50 | | | |
| Phenylphosphinic Acid | | 1.50 | | |
| Dimethyl Phosphite | | | 1.50 | |
| Pyrophosphoric Acid | | | | 0.75 |
| REACTION CONDITIONS | | | | |
| Temperature °C | 150 | 150 | 150 | 150 |
| Pressure Psig/KPa | 50/444.7 | 50/444.7 | 50/444.7 | 50/444.7 |
| Addition Time Hr | 5.58 | 6.08 | 6.50 | 2.40 |
| PRODUCT | | | | |
| # Ave. MW | 502.00 | 485.00 | 510.00 | 497.00 |
| # Mole EO | 6.93 | 6.54 | 7.11 | 6.82 |
| PEG % | 2.61 | 8.67 | 2.45 | 1.98 |
| Peak +/-2EO Wt% | 69.10 | 65.10 | 65.40 | 74.11 |
| OLIGOMERS | | | | |
| 0EO | 0.12 | 0.33 | 0.13 | 0.35 |
| 1EO | 0.32 | 0.64 | 0.36 | 0.32 |
| 2EO | 1.07 | 1.91 | 1.17 | 0.80 |
| 3EO | 3.10 | 4.83 | 3.06 | 2.57 |
| 4EO | 6.47 | 8.27 | 5.94 | 5.97 |
| 5EO | 10.36 | 11.66 | 9.35 | 11.05 |
| 6EO | 14.38 | 14.50 | 13.03 | 16.15 |
| 7EO | 16.22 | 14.82 | 15.02 | 18.26 |
| 8EO | 15.45 | 13.21 | 14.78 | 16.47 |
| 9EO | 12.73 | 10.94 | 13.20 | 12.17 |
| 10EO | 8.76 | 7.37 | 9.57 | 7.46 |
| 11EO | 5.43 | 4.72 | 6.44 | 4.16 |
| 12EO | 2.95 | 2.69 | 3.82 | 1.96 |
| 13EO | 1.44 | 1.51 | 2.12 | 1.02 |
| 14EO | 0.68 | 0.94 | 1.08 | 0.76 |
| 15EO | 0.32 | 0.63 | 0.52 | 0.50 |
| 16EO | 0.19 | 0.53 | 0.28 | |
| 17EO | | 0.47 | 0.11 | |
| 18EO | | | | |
| 19EO | | | | |
| 20EO | | | | |

TABLE 7.

| POISSON DISTRIBUTION | | | | | |
|---|---|---|---|---|---|
| PRODUCT | | | | | |
| # Mole EO | 2.00 | 3.50 | 6.00 | 6.50 | 7.00 |
| Peak +/-2EO Wt% | 92.00 | 81.13 | 68.80 | 66.90 | 65.10 |
| OLIGOMERS | | | | | |
| 0EO | 9.40 | 1.70 | 0.10 | 0.10 | |
| 1EO | 22.90 | 7.27 | 0.80 | 0.50 | 0.30 |
| 2EO | 27.10 | 15.03 | 2.80 | 1.90 | 1.30 |
| 3EO | 20.80 | 20.23 | 6.40 | 4.70 | 3.40 |
| 4EO | 11.80 | 20.06 | 10.80 | 8.60 | 6.70 |
| 5EO | 5.30 | 15.69 | 14.50 | 12.60 | 10.60 |
| 6EO | 1.90 | 10.12 | 16.10 | 15.00 | 13.60 |
| 7EO | 0.60 | 5.54 | 15.10 | 15.30 | 14.90 |
| 8EO | 0.20 | 2.63 | 12.30 | 13.50 | 14.20 |
| 9EO | | 1.11 | 8.90 | 10.50 | 11.90 |
| 10EO | | 0.42 | 5.70 | 7.40 | 8.90 |
| 11EO | | 0.14 | 3.30 | 4.60 | 6.10 |
| 12EO | | 0.04 | 1.80 | 2.70 | 3.80 |
| 13EO | | 0.01 | 0.90 | 1.40 | 2.20 |
| 14EO | | | 0.40 | 0.70 | 1.10 |
| 15EO | | | 0.20 | 0.30 | 0.60 |
| 16EO | | | 0.10 | 0.10 | 0.30 |
| 17EO | | | | 0.10 | 0.10 |
| 18EO | | | | | |

**Claims**

1. A method for the oxyalkylation of a linear or branched monohydric alcohol phenol, or an alkyl phenol having 6 to 30 carbon atoms with an alkylene oxide having 2 to 4 carbon atoms comprising reacting said monohydric alcohol and alkylene oxide in the presence of a catalyst, characterised in that the catalyst is a magnesium aryloxide or the reaction product of a magnesium aryloxide or high molecular weight magnesium alkoxide with a phosphorus-containing acid or ester.

2. A method according to Claim 1 characterized in that the magnesium aryloxide is derived from phenol or an alkylphenol.

3. A method according to Claim 2 characterized in that the alkylphenol is nonylphenol, dodecylphenol or dinonylphenol.

4. A method according to Claim 1 characterized in that the high molecular weight alkoxide is derived from an alcohol having 6 to 30 carbon atoms.

5. A method according to any one of Claims 1 to 4 characterized in that the magnesium aryloxide or alkoxide is obtained by mixing magnesium with a mixture of (i) a low molecular weight alcohol and (ii) phenol or alkylphenol or a high molecular weight alcohol, and subsequently removing the low molecular weight alcohol.

6. A method according to any one of Claims 1 to 4 characterized in that magnesium aryloxide is obtained by mixing magnesium with a mixture of a low molecular weight alcohol and a high molecular weight alcohol, and removing low molecular weight alcohol to leave the magnesium alkoxide of the high molecular weight alcohol, and reacting the said alkoxide with phenol or an alkylphenol.

7. A method according to any one of Claims 1 to 6 characterized in that the phosphorus-containing acid or ester is orthophosphoric acid, pyrophosphoric acid, an alkyl- or aryl - substituted phosphoric or phosphinic acid, or a $C_1$ - $C_4$ alkyl ester of one of said acids.

European Patent Office

# EUROPEAN SEARCH REPORT

Application Number

EP 89 30 5498

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
| --- | --- | --- | --- |
| D,A | EP-A-0 082 569 (SHELL INTERNATIONALE RESEARCH)<br>* Claims; page 5, lines 9-25 * | 1-7 | C 07 C 43/11<br>C 07 C 41/03 |
| A | EP-A-0 082 554 (SHELL INTERNATIONALE RESEARCH)<br>* Claims; page 6, line 28 - page 7, line 10 * | 1-7 | |
| A | EP-A-0 092 256 (UNION CARBIDE)<br>* Claims * | 1-7 | |
| D,A | US-A-4 721 817 (C.L. EDWARDS)<br>* Claims * | 1-7 | |
| D,A | US-A-4 239 917 (K. YANG)<br>* Abstract * | 1-7 | |

TECHNICAL FIELDS SEARCHED (Int. Cl.4)

C 07 C 41/00
C 07 C 43/00

The present search report has been drawn up for all claims

| Place of search | Date of completion of the search | Examiner |
| --- | --- | --- |
| THE HAGUE | 19-09-1989 | WRIGHT M.W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P0401)